(19) Europäisches Patentamt
European Patent Office
Office européen des brevets

(11) Publication number: **0 128 179**
**B1**

(12) **EUROPEAN PATENT SPECIFICATION**

(45) Date of publication of patent specification: **16.09.87**

(51) Int. Cl.⁴: **C 12 Q 1/38**

(21) Application number: **84900057.5**

(22) Date of filing: **14.12.83**

(86) International application number:
**PCT/FI83/00079**

(87) International publication number:
**WO 84/02352 21.06.84 Gazette 84/15**

# (54) METHOD FOR THE DETECTION OF INFLAMMATION OF THE UDDER.

(30) Priority: **17.12.82 FI 824348**

(43) Date of publication of application:
**19.12.84 Bulletin 84/51**

(45) Publication of the grant of the patent:
**16.09.87 Bulletin 87/38**

(84) Designated Contracting States:
**AT BE CH DE FR GB LI LU NL SE**

(56) References cited:
**EP-A-0 025 190**

**METHODS IN ENZYMOLOGY, Vol. II (1955), page 33**
**ENZYME HANDBOOK, Vol. II (1969), pages 618-9**

**J. Dairy Res. Vol. 48(2), pp. 213-23, publ. 1981, (HONKANEN-BUZALSKI I. SANDHOLM M) Trypsin-inhibitors in mastitic milk and colostrum: correlation between trypsin-inhibitor capacity, bovine serum albumin and somatic cell contents**

(73) Proprietor: **LABSYSTEMS OY**
**Pulttitie 9**
**SF-00810 Helsinki 81 (FI)**
(73) Proprietor: **EFLAB OY**
**Pulttitie 9**
**F-00810 Helsinki 81 (FI)**

(72) Inventor: **SANDHOLM, Markus**
**Airotie 1 D**
**SF-00830 Helsinki 83 (FI)**

(74) Representative: **Freed, Arthur Woolf et al**
**Reginald W. Barker & Co. 13 Charterhouse Square**
**London EC1M 6BA (GB)**

(56) References cited:
**Acta vet-scand. vol. 22(3-4), pp. 360-68, publ. 1981 (HONKANEN-BUZALSKI T. et al.) Milk antitrypsin activity during clinical and experimental bovine mastitis**

**Methods of enzymatic analysis, vol. 2, pp. 1018-20 and 1064-71, publ. 1974 by Verlag Chemie Weinheim, 2nd Ed. Edited Bergmeyer H.U.**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 128 179 B1

(56) References cited:

**Chemical Abstract, vol. 98(1983), abstracts no. 3332b and 33303c, (J. DAIRY)Res. 1982, 49(4), 577-85 and 587-96, date of publication of the articles not available to the examiner**
**Chemical Abstracts, vol. 81)1974), abstract no. 102616v, Veterinariya (Moscow) 1974, (3), 73-4**

**Description**

The present invention is concerned with a method for quantitative determination of inflammation of the udder so that the trypsin-inactivating effect of milk is measured, which effect has increased in connection with the inflammation of the udder. The reason of the said increase is mainly that the $\alpha_1$-antitrypsin in milk flows from the blood into the milk chamber in the case of inflammation of the udder.

The most important methods currently used for determining and detecting inflammation of the udder are based on bacteriology and on the determination of the number of cells out of a milk sample.

These methods have, e.g., the following drawbacks:

— The apparatuses for the determination and counting of the cells are expensive, laborious to use, and require abundant maintenance work. Under these circumstances, the present-day apparatuses are available only at the largest research laboratories of the dairy industry (e.g., Valio, Helsinki).

— The keeping and storage of the milk samples for the counting and determination of the cells is technically inconvenient and requires special operations.

— Depending on the time and mode of taking of the sample, the number of cells measured out of the milk sample is also influenced by other factors, besides the inflammation of the udder.

Among other things, out of the above reasons, by now, it has not been possible reliably to perform an examination teat by teat regarding the occurrence of inflammation of the udder. The detection of a latent inflammation of the udder has been highly uncertain. In different connections, it has been estimated that, e.g. in Finland, up to 40% of the entire cattle suffers from acute or latent inflammation of the udder. This again results in considerable economic losses.

A method for the quantitive determination in inflammation of the udder, by measuring the amount of trypsin-inhibitor present in udder milk, characterised by the following steps:

— a sample of test milk taken from the udder is clarified;

— a standard amount of trypsin is added to the sample;

— the proteolytic activity of the excess trypsin is determined by adding to the sample N-benzoylarginine-p-nitroanilide, from which trypsin liberates a coloured compound, on measuring the amount of the coloured compound as a change in optical density by using a vertical measurement beam.

The method of the present invention for the measurement of inflammation of the udder is simple, easy to use, and of favourable cost, as compared with the prior art methods. Moreover, the method has a high capacity, whereby a systematic examination of the cows individual by individual and teat by teat and a follow-up of the disease are possible. In the method of the invention, the new mode of pre-treatment of the samples is also considerably simpler as compared with prior art.

The method is based on the following basic observation: When a cow is taken ill with inflammation of the udder, the permeability of the wall cells of the capillaries at the area of inflammation is changed. As a result of this, in connection with inflammation of the udder, proteins of blood (in particular small-molecular ones) flow into the milk. So, it has been noticed that in connection with inflammation of the udder, e.g. blood albumin and $a_1$-antitrypsin are found in milk, which said compounds belong to the group of small-molecular proteins in blood. The presence of antitrypsin in milk in connection with inflammation of the udder is described, e.g. in the following papers: T. Honkanen-Buzalski, T. Katila, and M. Sandholm, "Milk antitrypsin activity during clinical and experimental bovine mastitis", *Acta Vet. Scand.* 1981 22.360—368; T. Honkanen-Buzalski and M. Sandholm, "Trypsin inhibitors in mastic milk and colostrum; correlation between trypsin inhibitor capacity, bovine serum albumin and somatic cell contents", *J. Dairy Res.* 1981 48, 213—223; and M. Sandholm, T. Honkanen-Buzalski, and R. Kangasniemi, "Milk trypsin inhibitor capacity as an indicator of bovine mastitis, a novel principle which can be automated" (Manuscript). It is on the basis of this observation that the present method, suitable for automation, has been developed.

Thus, the occurrence both of blood albumin and of $\alpha_1$-antitrypsin in milk, in principle, expresses inflammation of the udder, and the concentration correlates with the seriousness of the inflammation.

In practice, determination of blood albumin from milk is laborious and requires, among other things, immunochemical methods, but the determination of $\alpha_1$-antitrypsin is very easy, because it can be carried out enzymatically by measuring the protease-inhibitor capacity of milk.

This is why the method of the determination of inflammation of the udder, to be described below, has been developed, based on the determination of antitrypsin out of milk samples exactly on the basis of the said protease-inhibitor capacity.

During the development of the method, it has been kept in mind in particular that the method must be suitable for the treatment of large numbers of samples. In the simplest way, the activity of the protease-inhibitor can be measured by mixing trypsin into the sample and by measuring the proteolytic activity of the excess trypsin BAPNA (N-benzoylarginine-p-nitroanilide).

The invention will be described in more detail in the following with reference to the attached drawings in accordance with an exemplifying embodiment of the invention, in which drawings Figure 1 is a top view of a so-called microtiter disc used in the method of the invention and Figure 2 is a graphical presentation of measurement results obtained by means of the method of the invention.

Pretreatment of samples: Milk samples of 1 ml are clarified by means of rennin, 0.1 ml, which was in advance diluted to 1/50,000 by using a 0.1 M acetate buffer, pH 5.0. The clarification takes place by incubating the mixture of milk and rennin for 30 min at 37°C and by centrifuging or filtering the samples.

For the measurement of the trypsin-inhibitor content, 0.1 ml of the clarified milk samples is used, and the measurement is carried out as follows; The clarified milk samples are dosed into pits of the microtiter disc. Hereupon, into the pits is added 0.1 ml of standard trypsin solution (2.5 µg/ml, as dissolved in 1mM HCl) and 0.1 ml of substrate solution containing 1 mg N-benzoylarginine-p-nitroanilide (BAPNA) in 1 mg distilled water.

One milk sample is treated in each pit of the microtiter disc. Moreover, for the determination of the background values, there is an empty row of a so-called "blank-row" in the microtiter disc, as well as a series of standard dilutions (1, 1/2, 1/4 . . . 1/64) of the milk mixed by the dairies, representing the average quality of milk, and, moreover, a 100% trypsin control.

It has been noticed that the quantity of trypsin-inhibitor in milk is proportional to the degree of the inflammation of the udder, i.e. the quantity indicates the degree.

Under these circumstances, the idea with the use of the milk mixed by the dairies (which represents the average degree of health of the cows) as a series of standard dilutions is that the results in respect of each individual can also be given by comparing with the series of standard dilutions, i.e. by stating whether the individual concerned is more healthy or less healthy as compared with the average. This facilitates the standardization of the method considerably.

After addition of the reagents, the microtiter disc or the FP cuvette block (Labsystems Oy, Helsinki, Finland) is incubated for 3.5 hours, whereupon the quantity of yellow colour formed in the pits is measured by means of Titertek Multiskan (Eflab Oy, Helsinki, Finland) or FP—901 Analyzer (Labsystems Oy, Helsinki, Finland), both of them operating by means of the so-called principle of vertical measurement, the said apparatuses being described, e.g., in the following papers and patents:

Suovaniemi, O., "Performance and properties of the Finnpipette Analyzer System", Proceedings of the Second *National Meeting on Biophysics and Biotechnology in* Finland, Kairento, A. L., Riihimäki, E., and Tarkka, P., Eds., pp. 183—187 (1976);

Suovaniemi, O., "Method for Improving the Rate and Measurement Accuracy of Chemical Analysis", US Patent No. 4,144,030.

Suovaniemi, O., Järnefelt, J., "Discrete Multichannel Analysis Systems", *International Laboratory,* April 1982 and *American Laboratory,* June 1982; as well as Souvaniemi, O., CA 1,031,193, FR 7,442,147, GB 1,542,526, 1,392,792, US 4,144,030 (4,147,752 patents.

Figure 1 illustrates the arrangement of the samples on the microtiter disc. Reference numeral 1 denotes the samples, provided with current numbers 1 to 80. The series of standard dilutions (1/1, 1/2, 1/4 . . . 1/64) is denoted with numeral 2. Numeral 3' denote the 100% trypsin control (no milk sample), and numeral 4 the blank row. The determination of the trypsin-inhibitor out of a milk sample is based on the following biochemical reaction.

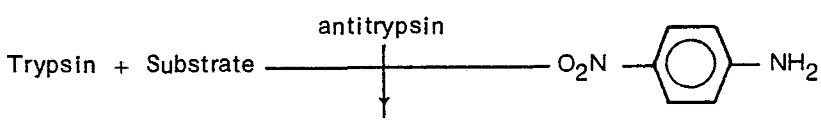


(product)
p—nitroanilin (coloured
compound, adsorption
at 405 nm)

In other words, the trypsin added to the pit of the microtiter disc decomposes the BAPNA (substrate) to a coloured compound. The antitrypsin derived from the milk inhibits this reaction by inactivating the trypsin.

The more trypsin-inhibitor is contained in a milk sample, the more efficiently does it prevent the activity of trypsin and, consequently, the generation of colour.

Thus, the presence of trypsin-inhibitor in a milk sample is inversely proportional to the strength of the colour to be measured.

As it has been noticed earlier that the concentration of trypsin-inhibitor in milk is proportional to the degree of inflammation of the udder, it can be said, on the basis of the test arrangement described above, that the more efficiently the milk sample inhibits the formation of colour on the microtiter disc, the more serious is the inflammation concerned.

The situation is shown graphically in Fig. 2. Therein, curve 5 illustrates the results from "healthy" milk, curve 6 from the milk mixed in the dairy, curve 7 results from 1/40 diluted serum, and curve 8 from "udder inflammation milk".

During the development of the method, the conditions have been arranged such that for the individual milk samples it is enough when only one dilution is performed within the range of 1/4 to 1/10 and the obtained result is compared with the standard curve obtained with the milk mixed in the dairy (compare the graph).

Under the selected test conditions, it is exactly this dilution that most clearly shows the differences between unhealthy and healthy cows.

It is to be noticed that the milk mixed by the dairies (in this case from about 5,000 cows), on the basis of the measurement results (cf. the graph), contains more antitrypsin than the milk obtained from a healthy cow. This is also understandable at present if one keeps in mind that about 40% of the cows are suffering from inflammation of the udder, part of them latent, and that, at present, there is no way for systematic observation of the cows teat by teat and individual by individual.

Under the selection conditions, on one microtiter disc, it is possible to treat eighty (80) milk samples. During one working day, by means of the said method, one person can process about 1,000 samples and produce the outputs from them.

The processing of the results is facilitated in particular by the circumstances that the disc reader is connected to a computer which is in advance programmed for processing the milk samples to be measured and for producing the outputs from them.

The first rather extensive study by means of the method described here was carried out by performing measurements of milk samples of 1,029 cows.

By means of the method described, it was noticed that the accuracy of measurement and the reproducibility were about 6% from the average. This figure also includes the errors that come from differences between microtiter discs as well as from variations from day to day.

The results obtained in the tests were in good correlation with two other modes of measurement of inflammation of the udder (measurement of albumin in milk as well as counting of cells).

When the method based on the measurement of antitrypsin is compared with other methods in use (e.g., measurement of albumin in or counting of cells), a highly remarkable additional advantage is obtained by means of measurement of antitrypsin: the pre-treatment of the samples is easy, because they can be preserved and stored either by to the samples adding sodium azide (bacteriostatic compound) or by freezing the samples. In the case of antitrypsin (in contrast to the other methods), the said operations do not interfere with the measurement.

Facilitated pre-treatment is of essential importance in order that it should be possible to treat large numbers of samples. This also permits observation animal by animal and teat by teat.

## Claims

1. A method for the quantitive determination of inflammation of the udder, by measuring the amount of trypsin-inhibitor present in udder milk, characterised by the following steps:
— a sample of test milk taken from the udder is clarified;
— a standard amount of trypsin is added to the sample;
— the proteolytic activity of the excess trypsin is determined by adding to the sample N-benzoylarginine-p-nitroanilide, from which trypsin liberates a coloured compound, and measuring the amount of the coloured compound as a change in optical density by using a vertical measurement beam.

2. A method as claimed in Claim 1 in which the contents of trypsin-inhibitor present in a test milk sample is determined; characterised in that, a reference sample of milk is taken from milk mixed by a dairy; the content of trypsin-inhibitor present in the reference sample is determined; and the trypsin-inhibitor content of the test sample is compared with that of the dairy reference sample, and the difference in measurement is related to the health of the animal from which the test sample is taken.

## Patentansprüche

1. Ein Verfahren für die quantitative Bestimmung der Entzündung des Euters, wobei die Menge des in der Eutermilch enthaltenen Trypsin-Inhibitors gemessen wird, gekennzeichnet durch die folgenden Schitte:
— eine Probe der dem Euter entnommenen Testmilch wird geklärt;
— eine Standardmenge an Trypsin wird der Probe zugegeben;
— die proteolytische Aktivität des Überschüssigen Trypsins wird durch Zugabe von N-benzoylarginin-p-nitroanilid vom dem das Trypsin eine gefärbte Verbindung freisetzt, zur Probe als Änderung der optischen Dichte unter Verwendung eines vertikalen Meßstrahls bestimmt.

2. Ein Verfahren wie in Anspruch 1 beansprucht, wobei die in einer Testmilchprobe enthaltenen Mengen eines Trypsininhibitors bestimmt werden, dadurch gekennzeichnet, daß eine Referenzprobe der Milch gemonnen wird, die in einer Molkerei vermischt wurde; der Gehalt des in der Referenzprobe vorliegenden Trypsininhibitors bestimmt wird; und der Gehalt an Trypsininhibitor der Testprobe mit jenem der Molkerei-Referenzprobe verglichen wird und die Meßdifferenz mit der Gesundheit des Tieres, von dem die Testprobe abgenommen wurde in Bezug gebracht wird.

**Revendications**

1. Procédé de détermination quantitative de l'inflammation du pis, en mesurant la quantité d'inhibiteur de la trypsine présente dans le lait du pis, caractérisé par les étapes suivantes:
— un échantillon de lait à essayer prélevé dans le pis est clarifié;
— une quantité normalisée de trypsine est ajoutée à l'échantillon;
— l'activité protéolytique de la trypsine en excès est déterminée en ajoutant à l'échantillon du N-benzoylarginine-p-nitroanilide, à partir duquel la trypsine libère un composé coloré et en mesurant la quantité du dit composé coloré par la modification de densité optique obtenue en utilisant un faisceau de mesure vertical.

2. Procédé selon la revendication 1, dans lequel la teneur en inhibiteur de la trypsine d'un échantillon de lait essayé est déterminée; caractérisé par le fait qu'un échantillon de référence du lait est prélevé dans la lait mélangé par un laiterie; la teneur en inhibiteur de la trypsine présent dans l'échantillon de référence est déterminée; et la teneur en inhibiteur de la trypsine de l'échantillon d'essai est comparée avec celle de l'échantillon de référence de laiterie, et la différence de mesure est mise en relation avec la santé de l'animal sur lequel l'echantillon d'essai est prélevé.

Fig.1.

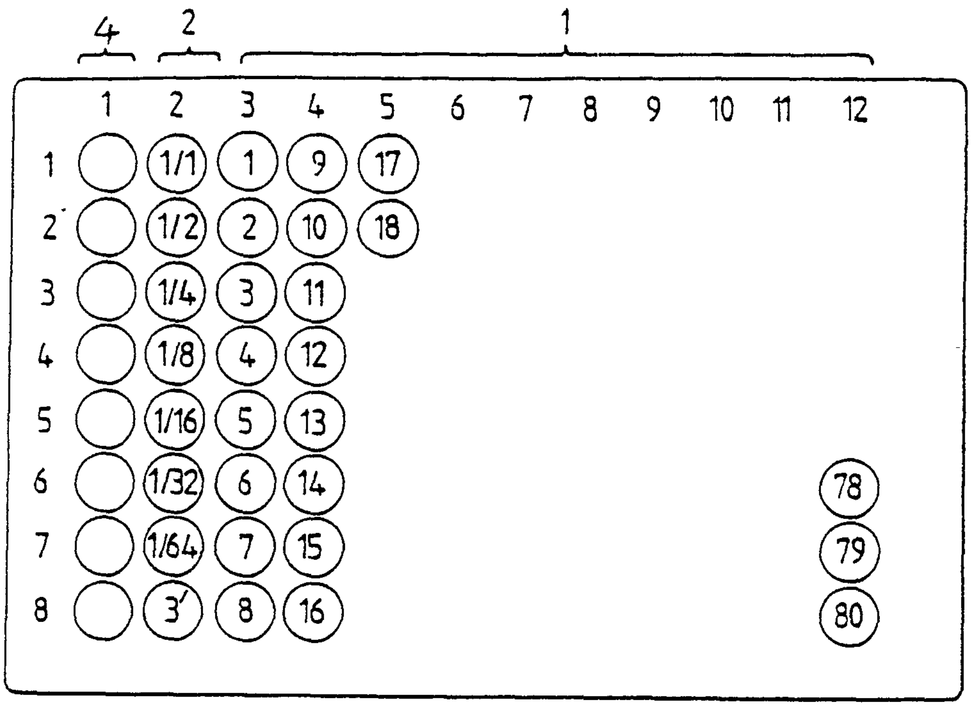
4
2
1
1 2 3 4 5 6 7 8 9 10 11 12
1/1
1/2
1/4
1/8
1/16
1/32
1/64
3'
78
79
80

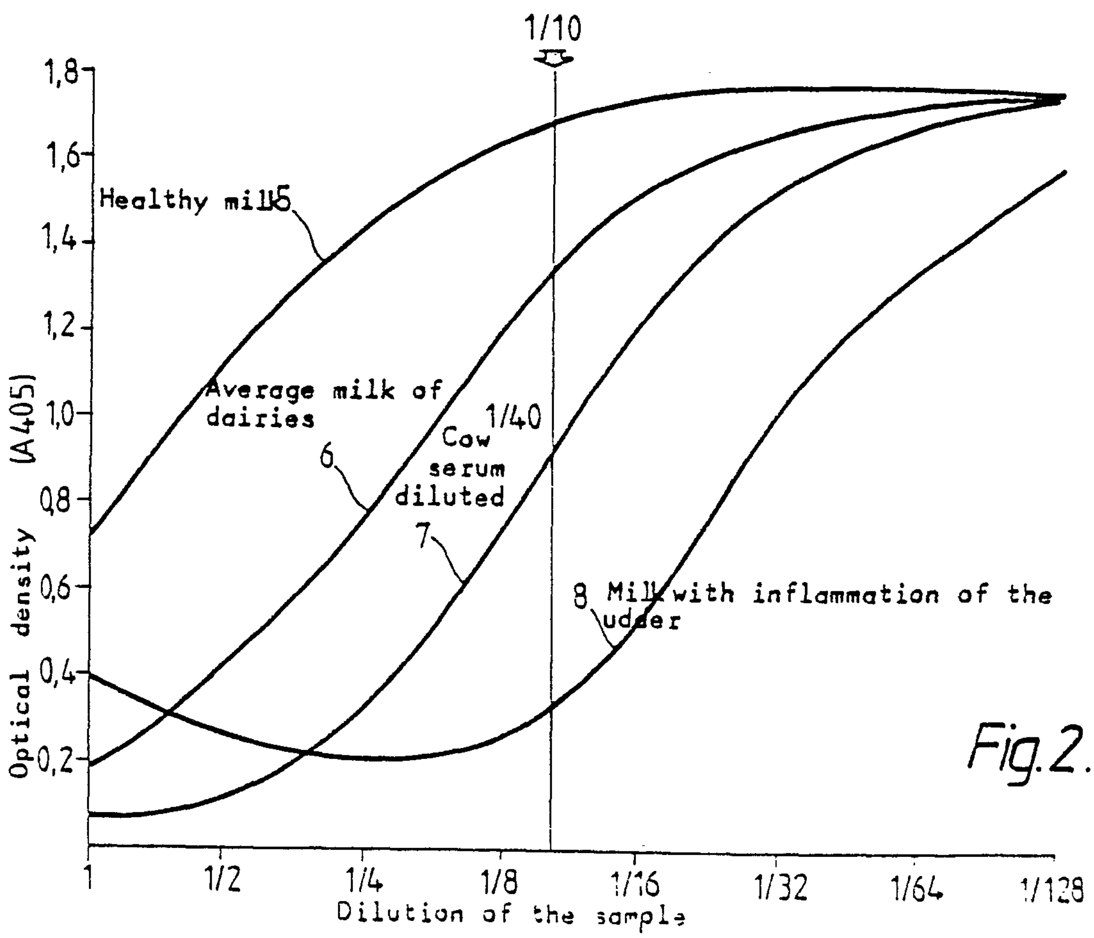
1/10
1/40
Healthy milk 5
Average milk of dairies
6
Cow serum diluted
7
8 Milk with inflammation of the udder
Optical density (A405)
1,8
1,6
1,4
1,2
1,0
0,8
0,6
0,4
0,2
1
1/2
1/4
1/8
1/16
1/32
1/64
1/128
Dilution of the sample

Fig.2.